# EUROPEAN PATENT SPECIFICATION

(11) **EP 0 434 744 B1**
(45) Date of publication and mention of the grant of the patent: **13.11.1996**
(21) Application number: 89910749.4
(22) Date of filing: 05.09.1989
(51) Int. Cl.: C07K 14/47, C07K 2/00

(54) **SERUM IMMUNOREGULATORY POLYPEPTIDE AND USES THEREFOR**
AUS SERUM ISOLIERTE UND DAS IMMUNSYSTEM BEEINFLUSSENDE POLYPEPTIDE UND DEREN VERWENDUNG
POLYPEPTIDE IMMUNOREGULATEUR DE SERUM ET SES EMPLOIS

(30) Priority: 09.09.1988 US 242739
(43) Date of publication of application: 03.07.1991
(73) Proprietor: THE REGENTS OF THE UNIVERSITY OF CALIFORNIA, Berkeley, California 94720 (US)
(72) Inventor: Chang, Yi-Han, Los Angeles, CA 90077 (US); Abraham, Edward, Malibu, CA 90265 (US)
(74) Representative: Silverman, Warren
(86) International application number: US8903800
(87) International publication number: WO9002760

(56) References cited:
- WPIL/DERWENT, Derwent Publications Ltd, London, GB; & JP-A-62 164 470 (KANEGAFUCHI CHEM K.K.)
- CRITICAL CARE MEDICINE,VOL. 14, NO. 2, 1986, ABRAHAM, E. AND CHANG, Y.-H.

## Description

The present invention relates to serum immunoregulatory peptides and uses therefor.

Immunoregulatory agents are substances that regulate or modify the normal or aberrant immune response of a host animal. Such agents are extremely important in medical applications, specifically in diseases that are caused by an abnormal immune response, such as rheumatoid arthritis (RA), systemic lupus erythrematosis (SLE), AIDS, etc. They are also useful where it is desirable to suppress normal immune response, such as in organ transplants and other types of tissue or organ grafts.

Various immunoregulatory agents are known in the art, but all have significant disadvantages. Some examples are cyclophosphamide, methotrexate, 6-MP and cyclosporin A. These drugs suffer from various shortcomings such as toxic side-effects and a narrow therapeutic index that severely limits their therapeutic application.

Immune response can be modified in many ways. One of the most desirable approaches is to influence or modulate the activity of suppressor T-cells. Suppressor T-cells are an important class of lymphocyte that have an important role in the regulation of immune response. These cells play an important role in the development of immuno-tolerance. Malfunction of these cells is believed to play a role in the pathogenesis of autoimmune diseases, such as RA and SLE. These diseases are believed to be caused by diminished suppressor T-cell activity and a resultant overproduction of auto antibodies. This situation may be reversed by activation of suppressor T-cells. In organ transplants, activation of suppressor T-cells, with the consequential suppression of the immune response, helps promote tolerance and prevent rejection of transplanted organs by the recipient's immune system.

Therefore, it would be extremely advantageous to have an immunoregulatory agent that activates suppressor T-cells, thereby regulating immune response, but which has a wide therapuetic index and does not have toxic side-effects. The present invention satisfies this need and provides other related advantages.

Abraham, Edward and Chang, Yi-Han, Critical Care Medicine, vol. 14, 1986, pp. 81-86, discloses the isolation from haemorrhagic blood of fractions having molecular weights between 13,000 Da and 23,000 Da and having an immunosuppressive activity. Abraham, Edward, Tanaka, Traci and Chang, Yi-Han, Crit. Care Med., vol 16, 1988, pp. 307-311, discloses further immunosuppressive properties of the fractions having molecular weights between 13,000 Da and 23,000 Da.

According to the present invention, there is provided a serum immunoregulatory polypeptide (SIP) isolatable from mammalian haemorrhagic blood which
(a) activates suppressor T-cells and thereby suppresses lymphocyte proliferation in mammals,
(b) suppresses lymphokine production, and
(c) has a molecular weight of 16,000 ± 2,000 Da or 29,000 ± 2,000 Da.

The present invention resides in the discovery of previously undescribed and unisolated relatively low molecular weight serum immunoregulatory polypeptides (SIPs) that are present in haemorrhagic mammalian blood. Different mammalian species appear to have different SIPS, and there may be more than one SIP in the blood of each mammalian species. The SIPs regulate the immune response in mammals and therefore have far reaching value in the treatment of many diseases, including allograft rejection, auto-immunity, graft versus host diseases, allergies, cancer and AIDS. One mechanism by which the SIPs function to regulate immune response is the activation of suppressor T-cells, resulting in suppression of lymphocyte proliferation. Additionally, the SIPs suppress production of lymphokines, including interleukin-2 (IL-2).

The SIPs of the present invention are low molecular weight compounds obtained from mammalian haemorrhagic serum. Haemorrhagic blood is blood obtained from mammals following haemorrhage. Haemorrhagic serum is obtained by allowing haemorrhagic blood to clot, followed by centrifuing and subsequent removal of supernatants. Haemorrhagic serum shows immunoregulatory activity where control samples of normal serum do not.

One SIP was obtained from haemorrhagic serum of rats. The hemorrhagic serum was shown to suppress phytohemagglutinin (PHA) induced proliferation of rat peripheral blood lymphocytes. The active fraction of the hemorrhagic serum was isolated, and activity of the serum showed that the active substance, the SIP, had a molecular weight of approximately 16,000. An SIP isolated from human hemorrhagic serum, and having similar biological activity, had a molecular weight of approximately 29,000.

An SIP of the present invention was also shown to suppress the development of cell-mediated and humoral immunity in vivo. SIP treated animals showed significantly lower cell-mediated and humoral immune response to EL₄ cells than control animals.

SIPs also suppress the proliferation of rat peripheral blood mononuclear cells (PBMC). Human SIP was found to suppress PHA-induced proliferation of rat PBMCs. Similarly, rat SIP suppressed PHA-induced proliferation of mouse spleen cells. It thus appears that there is a substantial degree of homology between structure and function of SIP from different species.

The implications of the SIPs of the present invention are far reaching and dramatic because they significantly affect the immune response.

Regarding auto-immune diseases, suppressor T-cells ordinarily block the development of auto-antibodies in auto-immune diseases. There is substantial evidence indicating that auto-immune diseases such as SLE and RA involve a loss of suppressor T-cell activity. Activation of suppressor T-cells using an SIP of the present invention, one mechanism by which the immune response is regulated, is useful in treating such diseases.

It has been firmly established that suppressor T-cells monitor and regulate the immune response. Suppressor T-cells serve teleologically as an "off-switch" for the immune response and play an important role in establishing tolerance. For example, it has been shown that suppressor T-cells can replace response with non response at very low levels of antigen. IL-2, on the other hand, has an opposite effect. Current evidence suggests that exposure to antigen together with appropriate MXC causes unresponsiveness, and that a second signal, IL-2, is required for immunity. Thus, IL-2 converts a "tolerogenic" stimulus into an immunogenic stimulus. By activating suppressor T-cells and decreasing the generation of IL-2, an SIP should promote the establishment of tolerance. This would make the SIPs of the present invention therapeutic in allograft rejection, graft versus host diseases, and in situations in which more subtle effects on the production of lymphokines may be important, such as in allergies or certain types of leukemias that are characterized by requiring IL-2 at certain stages.

Regarding therapeutic applications, it is possible that SIPs may play a role in the pathogenesis of certain immunodeficiency diseases. Monoclonal antibodies against these SIPs may be derived and used to enhance the immune response in such situations.

Preparing monoclonal antibodies directed against the SIPs also has diagnostic applications. Such antibodies would provide the means for measuring the blood levels of the SIPs, enabling the identification of subgroups of patients who are at particular risk of developing diseases of abnormal immune regulation or developing serious infections after trauma injury.

The following detailed description demonstrates, by way of example, the principles and uses of the present invention.

The present invention is embodied in previously undescribed and unisolated serum immunoregulatory polypeptides (SIPs) of molecular weight approximately 16,000 Da ± 2,000 Da and 29,000 Da ± 2,000 Da that modulate the immune response in mammals, at least in part by activating suppressor T-cells. The SIPs also suppress lymphocyte proliferation and lymphokine production, including interleukin-2, in mammals. The SIPs of the present invention can be obtained from the hemorrhagic blood of various mammals, although rats and a brain-dead human were used for experimentation purposes.

The SIPs of the present invention have dramatic potential for the effective treatment of a variety of conditions and diseases. For example, suppressor T-cells ordinarily block the development of auto-antibodies in auto-immune diseases, such as SLE and RA. The SIPs, by enhancing activation of these suppressor T-cells, could be useful in treating these diseases.

Treatment can be accomplished by various methods. In vitro, the SIP need simply come into physical contact with the suppressor T-cells. In vivo, administration of the SIP may be by various mechanisms that function to bring the SIP into physical contact with the supressor T-cells, including but not limited to subccutaneous injection, intravenous injection, intraperitoneal injection and delivery via an appropriate pharmaceutical vehicle. The same is true for any cells that are to be treated with an SIP.

Also, interleukin-2 has been known to convert a tolerogenic stimulus into an immunogenic stimulus. By activating suppressor T-cells and decreasing the generation of IL-2, the SIPs promotes tolerance, and would therefore have therapeutic use in allograft rejection cases, graft versus host diseases and other situations characterized by the requirement of IL-2 at various stages. As with suppressor T-cells, treatment can be effected in vitro or in vivo by suitable mechanism to bring the SIP into physical contact with the lymphocytes.

By preparing monoclonal antibodies directed against the SIPs, which can be done using conventional techniques known in the art, the level of these SIPs in blood can be accurately measured using conventional methods.

The SIPs are particularly advantageous because unlike other immunosuppressants, such as cyclosporin A, the SIPs are normal immunoregulatory proteins, and would be expected to have few significant side effects.

The SIPs may be used to treat cells both in vitro and in vivo. In both cases, an effective amount of SIP must be used. For in vitro treatment, 1 ug of SIP per ml of cell medium is effective, but a lower concentration may also be used, depending on the concentration of cells in the medium. Experimentation can be used to determine the lowest effective limit for various cell concentrations. The upper limit for effective treatment is an amount that will not overwhelm the cell medium with too much activity, also determinable by experimentation.

For in vivo treatment, .75 mg of SIP per 1 Kg of body weight of mammal I.P. has been found to be effective in mice, but the lowest effective limit may vary from mammal to mammal. This can be easily determined by experimentation. The upper effective limit is an amount of SIP, also determinable by experimentation, that will not cause so much biological activity in the animal so as to be harmful.

The SIPs of the present invention were obtained from the hemorrhagic serum of rats and a human, but the serum of other mammals, including but not limited to rabbits, cats, dogs, horses, sheep, monkeys, etc. may be used. Hemorrhagic blood is blood obtained from mammals following hemorrhage. Hemorrhagic serum is obtained by allowing hemorrhagic blood to clot, followed by centrifuging and subsequent removal of supernatants.

Example 1 demonstrates how hemorrhagic serum, containing one of the SIPs of the present invention, was obtained from rats. In order to obtain hemorrhagic serum, 30% of the rats' total blood volume was bled from each animal over a period of ten minutes. The animals were permitted to recover for two hours, and additional blood samples were taken. These second blood samples constitute hemorrhagic blood, which has been produced by the animal's system after a major hemorrhage (in this case, loss of 30% of total blood volume). Hemorrhagic serum was then obtained by clotting and removal of supernatants. Human hemorrhagic serum was similarly obtained from a brain-dead patient.

It is important to note that hemorrhagic serum can be obtained using other parameters for percent of total blood volume bled and waiting time after blood loss before removing hemorrhagic blood. Although 30% of total blood volume was removed in Example 1, hemorrhagic blood can be obtained by removing as little as 1% or as much as 70% of total blood volume (more than 70% would kill the host animal prior to any opportunity to take a second blood sample). Ten percent to forty percent is preferred. Similarly, although two hours elapsed after blood volume loss before removing hemorrhagic blood, as little as a 15 minute time period or as much as 72 hours after initial blood volume removal will also suffice. Thirty minutes to two hours is preferred.

The particular method of isolation of the SIPs of the present invention from hemorrhagic blood is not critical. Isolation by chromatography is preferred, including gel elecrophoresis or isoelectric focusing (IEF) followed by eluting the SIPs from a gel. Other isolation techniques are known to those skilled in the art and may also be used.

The following examples serve to illustrate tne present invention.

### EXAMPLE 1

### Obtaining of Hemorrhagic Serum

The carotid artery of pentobarbital anesthetized rats is catheterized with PE 50 tubing. The tubing is tunneled through the back of the neck and secured with a jacket, tether and swivel apparatus. The rats are then placed in individual cages, given food and water ad libitum, and allowed to recuperate for a minimum of three days. The PHA-induced lymphocyte proliferation response of catheterized-recuperated animals is the same as in normal, uncatheterized control animals (1). To produce hemorrhagic serum, a volume of blood approximately equal to 30% of total blood volume, is bled first from each animal over a 10 minute period. Two hours later, the remaining blood is bled from each animal. Serum obtained from the second sample constitutes "hemorrhagic serum".

Human hemorrhagic serum was similarly obtained from a brain-dead patient before the removal of various organs for transplantation.

### EXAMPLE 2

### Effect of Hemorrhagic Serum on Mitogen-Induced Lymphocyte Proliferation

Procedures as set forth in Example 1 were used to obtain hemorrhagic serum, but with different blood volume loss and waiting periods.

When lymphocytes from normal rats (n = 28) were incubated with PHA (125 ug/ml) in the presence of pooled serum collected 2 hours after removal of 30% of total blood volume from three to five rats (hemorrhagic serum), there was a 44% decrease in proliferative response as compared to that present when lymphocytes from the same animal were incubated in the presence of pooled normal serum: 10,084 ± 2108 cpm vs. 17,973 ± 2928 cpm, p .001.

Similarly, lymphocyte proliferative response was reduced when the cells were incubated (1) in the presence of serum collected two hours after removal of 5% of total blood volume and (2) in the presence of serum collected 15 minutes after removal of 30% of total blood volume. (See Table 1).

**Table 1**

| Group | Hemorrhage | | 3H uptake (cpmx10²±SEM) | P |
|---|---|---|---|---|
| | vol* | Time** | | |
| Normal (Control) | ----- | ----- | 87 ± 11 | 0.001 |
| Hemorrhaged | 5 | 120 | 39 ± 1 | |
| | | | | |
| Normal (Control) | ----- | ----- | 116 ± 4 | |
| Hemorrhaged | 30 | 15 | 81 ± 6 | 0.001 |

| | | | | |
|---|---|---|---|---|
| *Volume of hemorrhage, % of TBV | | | | |
| **Minutes post hemorrhage when blood sample was taken | | | | |

### EXAMPLE 3

### Isolation of SIP from Rat Hemorrhagic Serum

A serum immunoregulatory polypeptide was isolated from rat hemorrhagic serum by ammonium sulfate precipitation and purified sequentially by Sephadex G-75 chromatography, anion exchange HPLC, and reverse phase HPLC using a C18 column.

Electrophoretically homogeneous SIP can be also obtained by the following procedures:
(a) ammonium sulfate precipitation - DEAE Sephadex chromatography - Sephadex G-75 chromatography - reverse phase HPLC (C18).
(b) Amicon filtration - Anion exchange HPLC - Sephadex G-75 chromatography - reverse phase HPLC (C18).
Isolation of SIPs can be accomplished using various methods, with chromatography being preferred.

### EXAMPLE 4

### Control Phytohemagglutinin (PHA) Induced Lymphocyte Proliferation and Effect of SIP on PHA Induced Lymphocyte Proliferation

Phytohemagglutinin (PHA) induced proliferation of rat peripheral blood lymphocytes is assessed by modification of the technique of Keller et al. (2). Blood (approximately 7 ml) is collected from rats in heparinized syringes, diluted 1:6 with 0.9% saline, layered onto 70% Percoll.(Pharmacia, Piscataway, NJ) and centrifuged at 400 g for 30 minutes. The lymphocyte layers are harvested, washed 3 times with RPMI 1640 medium, supplemented with penicillin/streptomycin and adjusted to pH 7.3. Viability, as determined by trypan blue exclusion, is consistently greater than 95%. Lymphocyte purity is greater than 92% as determined by a non-specific esterase stain.

The washed lymphocytes are adjusted to 2.5 x 10⁶ cells/ml in RPMI 1640 medium supplemented with 5% pooled rat serum or 20% fetal calf serum, penicillin/streptomycin, at pH 7.3. Phytohemagglutinin (Difco, Detroit, MI) is added to the cell suspension to produce a final concentration of 125 ug/ml which we have previously found to produce maximal lymphocyte proliferation. Two fifth milliliter (200 ul) of the cell suspension (5 X 10⁵ cells) is placed into each well of a flat bottom microliter plate (Falcon 3=040 microliter plate, Oxnard, CA). All cultures are performed at least in triplicate. The cultures are incubated at 37°C in a humidified atmosphere at 5% CO₂. After 2 days of incubation, 0.2 uCi of 5-(I¹²⁵)-2-deoxyuridine (Amersham, Arlington Heights, IL) in 25 ul of 10⁻⁶ M 5-F-2-deoxyuridine (Sigma, St. Louis, MO) in RPMI 1640 is added to each culture. After 18 hours the cells are harvested onto glass fiber filter paper and radioactivity assayed in a gamma counter. Uptake of ¹²⁵I is calculated by determining the mean of the triplicate cultures.

The effect of SIPs on PHA-induced proliferation of peripheral blood mononuclear cells (PBMC) was studied in accordance with the techniques described above. The addition of rat SIP into rat PBMC cultures suppressed the proliferation of rat PBMC (control, 22362±2434; SIP, 13536±1645). A substantial degree of homology in structure and function exists between SIPs of different species. Human SIP suppressed PHA-induced proliferation of rat PBMCs (Control, 3974±244; human SIP, 423±145). Rat SIP suppressed PHA-induced proliferation of mouse spleen cells (Control, 94633±10390; rat SIP 65720±11808).

These results show a significant suppression, using an SIP, of the proliferation of rat peripheral blood mononucleus cells. Moreover, the results show substantial homology between SIPs of different species, including humans and rats, and rats and mice. This is of significant importance, as it could enable human lymphocyte proliferation to be controlled by an SIP obtained from the hemorrhagic serum of laboratory animals.

### EXAMPLE 5

### Determination of Molecular Weight of SIP Obtained From Rat Hemorrhagic Serum

The active fractions of SIP, i.e. fractions that suppressed PHA-induced lymphocyte proliferation in Example 4, obtained from anion exchange HPLC, which showed three bands corresponding to molecular weights of 14,000, 16,000 & 19,000, were further purified by reverse phase HPLC using a C18 column. SDS PAGE analysis of the peak activity fraction showed a single band corresponding to a molecular weight of approximately 16,000 with a possible variation of ± 2,000.

### EXAMPLE 6

### Determination of Molecular Weight of Human Hemorrhagic Serum SIP

A human hemorrhage-induced serum immunoregulatory polypeptide with a molecular weight of 29,000 (also with a possible variation of ± 2,000) was similarly isolated and purified from hemorrhagic serum obtained from a brain-dead patient in accordance with the procedures set forth in Examples 1-5.

Minute amounts of this SIP were detected recently in normal blood suggesting that it may be a normal immunoregulatory protein. Based on their molecular weight and biological activity, the SIPs of the present invention appear different from inhibitors of cell proliferation reported in the literature including contra-IL-2(6), a 30-100 kd inhibitor of mitogen-induced lymphocyte proliferation found in synovial fluid of patients with rheumatoid arthritis (7), a 70-kd factor which inhibits the production and action of IL-2 (8,9), a 25-kd inhibitor of IL-1 (10), a 97-kd inhibitor of the response to IL-2 of an IL-2 dependent cell line (11), suppressive factors in serum of patients after trauma and thermal injury (12-16), and others (17-27).

### EXAMPLE 7

### Effect of SIPs on the Development of Immune Response to EL₄ Cells

### a) ⁵¹Cr Labeling of EL₄ Cells

Both the humoral and cellular assays of immune response are based upon the lysis of ⁵¹Cr-labeled EL₄ cells. Labeling is carried out essentially according to the method of Canty and Wunderlich (3). To 1 X 10⁸ EL₄, cells in 20% fetal calf serum and Hank's minimum essential medium (HMEM) are added 60 uCi of ⁵¹Cr. The mixture is incubated for 30 minutes in 20% CO₂, at 37°C. The labeled cells are washed three times in HMEM with fetal calf serum to remove unbound ⁵¹Cr radiolabel and are resuspended in the same medium in a concentration of 1 X 10⁵ cell/ml.

### b) Cell-mediated Immunity to EL₄ Cells

Cell-mediated immunity to EL₄ cells is measured according to a previously reported procedure (4). Briefly, spleen cells are obtained from EL₄ cell injected or saline injected Lewis rats. The cells are washed and resuspended in HMEM (20% fetal calf serum) to a concentration of 3 X 10⁷ cells/ml. The spleen cell suspension (0.5ml) is mixed with 5 X 10⁴ labeled EL₄ cells (0.5 ml) in a 35 mm petri dish (Falcon Plastics) and incubated for 3 hours at 37™C in 10% CO₂ on a rocker platform making 8 reciprocations per minute. The amount of ⁵¹C released from the EL₄ cells is then determined. All assays are performed in triplicate.

### c) Humoral Immunity

Humoral immunity to EL₄ cells is measured by a procedure we have reported previously (4). Briefly, serum from Lewis rats is diluted in HMEM containing fetal calf serum (20%). A 0.2 ml portion of ⁵¹Cr labeled EL₄ cell suspension (1.25 X 10⁶ cells/ml) is mixed with 0.3 ml of a 1/6 dilution of rabbit complement (absorbed with 5 X 10⁸ EL₄ cells/ml) and then added to 0.5 ml of serial dilutions of rat sera. The dilution giving 50% lysis of EL₄ cells is determined from the van Krogh equation (5) to be the titer of the serum.

### d) Effect of SIPs

Effects of SIPs on the development of immune response to EL₄ cells were studied. Mice were given 25 ug of SIP or saline (control), I.P., immediately before and 3 days after immunization with 1 x 10⁸ EL₄ cells. Spleen lymphocytes and serum samples were harvested 7 days after immunization and were assayed for cell-mediated and humoral immunity according to a procedure we have reported previously (4). Cell-mediated cytotoxicity was assayed at a 300:1 effector:target cell ratio. Complement-dependent antibody mediated lysis of target cells was assayed at a 100 x dilution of serum. Results (Table 2) are expressed as mean ± SEM. The data show that SIP treated animals have significantly lower cell-mediated as well as humoral immune response than the controls.

**TABLE 2**

| Treatment | Cell-Mediated Immunity (% lysis) | Humoral Immunity (% lysis) |
|---|---|---|
| Saline | 38.4 ± 2.8 | 36.3 ± 0.9 |
| SIP | 3.1 ± 2.6 | 27.6 ± 4.4 |

### REFERENCES

1. Abraham E, Chang Y-H: The effects of hemorrhage on lymphocyte proliferation. Circ Shock 15:141-49, 1985.
2. Keller SE, Schleifer SJ, McKegney MP, et al: A simplified method for assessing phA induced stimulation of rat peripheral blood lymphocytes. J Immunol Methods 51:287-91, 1982.
3. Canty TG, Wunderlich JR: Quantitative in vitro assay of cytotoxic cellular immunity. J Nat Cancer Inst. 45:761, 1970.
4. Chang Y-H: Adjuvant polyarthritis I: Incorporation of quantitative measures of humoral and cellular immune response. J Pharmacol Exp Ther. 201:1-7, 1977.
5. Kabat EA, Mayer MM: Experimental immunochemistry. Springfield Illinois, Charles C. Thomas, 1961, p 136.
6. Spiegel, J.P., Djew, Jy, Stock, N.I., Masur H., Gelmann E.P., Quinnan, G.V.,Jr. (1985) Sera from patients with acquired immunodeficiency syndrome inhibit production of interleukin-2 by normal lymphocytes. J.Clin.Invest. 75, 1957-1964.
7. Zembala, M., Lemmel, E-M (1980) Inhibitory factor(s) of lymphoproliferation produced by synovial fluid mononuclear cells from rheumatoid arthritis patients:the role of monocytes in suppression. J Immunol 125, 1087-1092.
8. Krakauer, T.(1985) A macrophage derived factor that inhibits the production and action on interleukin 2. J. Leukocyte Biol 3,429-440.
9. Miossec, P., Kashiwado, T., and Ziff, M. (1987) Inhibitor of interleukin-2 in rheumatoid synovial fluid. Arth & Rheum 30, 121.
10. Arend, W.P., Joslin, F.G., Massoni, R.J. (1985) Effects of the immune complexes on production by human monocytes of interleukin 1 or an interleukin 1 inhibitor. J. Immunol 134, 3868-3875.
11. Fontana, A., Hengartner, H., deTRibolet, N., Weber, E. (1984) Glioblastoma cells release interleukin 1 and factors inhibiting interleukin 1-mediated effects. J. Immunol. 132,1837-1844.
12. Ninneman, J. L. (1980) Immunosuppression following thermal injury through B cell activation of suppressor T cells. J Trauma 20,206-13.
13. Constantian, M.B., Menzoian, Nimberg, R.B., Schmid, K., Mannick, J.A. (1977) Association of a circulating immunosuppressive polypeptide with operative and accidental trauma. Ann Surg 185,73-9.
14. Constantian, M.B. (1978) Association of sepsis with an immunosuppressive polypeptide in the serum of burn patients. Ann Surg 188,209-15.
15. Ninneman, J.L., Fisher, J.C. Wachtel, T.L. Thermal injury-associated immunosuppression occurrence and in vitro blocking effect of post-recovery serum. J Immunol. 122,1736-41.
16. Ninneman, J.L., Condie, J.T., Davis, S.E., Crockett, R.A. (1982) Isolation of immunosuppressive serum components following thermal injury. J Trauma. 22,834-44.
17. Aune, T.M., Webb, D.R., Pierce, C.W. (1983) Purification and initial characterization of the lymphokine, soluble immune response suppressor. J Immunol. 131,2848-54.
18. Thomas, D.W., Roberts, W.K., Talmage, D.W. (1975) Regulation of the immune response: production of a soluble suppressor by immune spleen cells in vitro. J. Immunol. 114,1616-21.
19. Namba, Y., Waksman, B.H. (1975) Regulatory substances produced by lymphocytes. I. Inhibitor of DNA synthesis in the rat. Inflammation 1,5-12.
20. Lee, S.C., Lucas, Z.J. (1977) Regulator factors produced by lymphocytes. II. Inhibition of cellular DNA synthesis is associated with a factor inhibiting DNA polymerase-alpha activity. J Immunol. 118,88-95.
21. Chiba, K., Nishimura, T., Hashimoto, Y. (1985) Stimulated rat T cell derived inhibitory factor for cellular DNA synthesis (STIF). J Immunol. 134,1019-27.
22. Namba, Y., Waksman, B.H. (1985) Regulatory substances produced by lymphocytes II. Lymphotoxin in the rat. J Immunol. 115,1018-14.
23. Greene, W.C., Fleisher, T.H., Waldmann, T.A. (1981) Soluble suppressor supernatants elaborated by concanavalin A-activated human mononuclear cells. I. Characterization of a soluble suppressor of T cell proliferation. J Immunol. 126,1185-90.
24. Payan, D.G., Hess, C.A., Goetzl, E.J. (1984) Inhibition by somatostatin of the proliferation of T-lymphocytes and Molt-4 lymphoblasts, Immunology 84,433-41.
25. Payan, D.G., Goetzl, E.J. (1985) Modulation of lymphoctyes function by sensory neuropeptides. J Immunol. 135,783-875.
26. Ottaway, C.A., Greenberg, G.R. (1984) Interaction of VIP with mouse lymphocytes: specific binding and the modulation of mitogen responses. J. Immunol. 132,417-24.
27. O'Dorisio, M.S., Wood, C. L., O'Dorisio, T.M.(1985) Vasoactive intestinal peptide and neuropeptide modulation of the immune response. J Immunol. 135,792s-98s.

It is apparent from the foregoing that the SIPs of the present invention are extremely effective in regulating the immune response in mammals, as well as in diagnostic applications. Such properties have far reaching and wide therapeutic and diagnostic applications.

## Claims

1. A serum immunoregulatory polypeptide (SIP) isolatable from mammalian haemorrhagic blood which
(a) activates suppressor T-cells and thereby suppresses lymphocyte proliferation in mammals,
(b) suppresses lymphokine production, and
(c) has a molecular weight of 16,000 ± 2,000 Da or 29,000 ± 2,000 Da.

2. A serum immunoregulatory polypeptide as claimed in claim 1, which is isolated from human haemorrhagic blood and which has a molecular weight of 29,000 ± 2,000 Da.

3. A serum immunoregulatory polypeptide as claimed in claim 1, which is isolated from rat haemorrhagic blood and which has a molecular weight of 16,000 ± 2,000 Da.

4. A serum immunoregulatory peptide as claimed in claim 1, 2 or 3, which is isolated from haemorrhagic blood which is removed from said mammal after between about 1% to about 70%, preferably between about 10% to about 40%, of said mammal's total blood volume has been removed and between about 5 minutes to about 72 hours, preferably between about 30 minutes to about 2 hours, has been permitted to elapse.

## Patentansprüche

1. Immunregulatorisches Serum-Polypeptid (SIP), das aus hämorrhagischem Säugetier-Blut isolierbar ist und
(a) die Suppressor-T-Zellen aktiviert und hierdurch bei Säugetieren die Lymphozyten-Proliferation unterdrückt,
(b) die Lymphokin-Bildung unterdrückt, und
(c) ein Molekulargewicht von 16,000 ± 2,000 Da oder 29,000 ± 2,000 Da hat.

2. Immunregulatorisches Serum-Polypeptid (SIP) nach Anspruch 1, das aus hämorrhagischem Menschen-Blut isoliert ist and ein Molekulargewicht von 29,000 ± 2,000 Da hat.

3. Immunregulatorisches Serum-Polypeptid (SIP) nach Anspruch 1, das aus hämorrhagischem Ratten-Blut isoliert ist und ein Molekulargewicht von 16,000 ± 2,000 Da hat.

4. Immunregulatorisches Serum-Polypeptid (SIP) nach Anspruch 1, 2 oder 3, das aus hämorrhagischem, einem Säuger entnommenen Blut isoliert ist und zwar nachdem zwischen etwa 1% und etwa 70%, vorzugsweise zwischen etwa 10% bis etwa 40% des gesamten Säuger-Blutvolumens entnommen wurde und man zwischen etwa 5 Minuten und etwa 72 Stunden, vorzugsweise zwischen etwa 30 Minuten und etwa 2 Stunden, verstreichen ließ.

## Revendications

1. Polypeptide immunorégulateur de sérum (PIS) pouvant être isolé du sang hémorragique de mammifère qui
(a) active les cellules T suppressives et par conséquent supprime la prolifération des lymphocytes chez les mammifères,
(b) supprime la production de lymphokine, et
(c) présente une masse moléculaire de 16 000 ± 2 000 Da ou 29 000 ± 2 000 Da.

2. Polypeptide immunorégulateur de sérum conforme à la revendication 1, que l'on isole du sang hémorragique humain et qui présente une masse moléculaire de 29 000 ± 2 000 Da.

3. Polypeptide immunorégulateur de sérum conforme à la revendication 1, que l'on isole du sang hémorragique de rat et qui présente une masse moléculaire de 16 000 ± 2 000 Da.

4. Polypeptide immunorégulateur de sérum conforme à la revendication 1, 2 ou 3, que l'on isole du sang hémorragique qui est éliminé dudit mammifère, après élimination d'environ 1 % à environ 70 %, de préférence d'environ 10 % à environ 40 % dudit volume total de sang de mammifère et après avoir laisser s'écouler un temps d'environ 5 minutes à environ 72 heures, de préférence d'environ 30 minutes à environ 2 heures.
